Europäisches Patentamt

European Patent Office

Office européen des brevets

(11) Publication number: **0 416 505 A2**

## (12) EUROPEAN PATENT APPLICATION

(21) Application number: 90116877.3

(22) Date of filing: 03.09.90

(51) Int. Cl.5: **C12N 15/31**, C12N 15/70, C12N 1/21, C12P 21/02

The microorganism(s) has (have) been deposited with Institute for Fermentation, Osaka, Japan under number 14933.

The applicant has subsequently filed a sequence listing and declared, that it includes no new matter.

(30) Priority: 04.09.89 JP 227439/89

(43) Date of publication of application:
**13.03.91 Bulletin 91/11**

(84) Designated Contracting States:
**AT BE CH DE DK ES FR GB GR IT LI LU NL SE**

(71) Applicant: **TAKEDA CHEMICAL INDUSTRIES, LTD.**
**3-6, Doshomachi 2-chome Chuo-ku**
**Osaka 541(JP)**

(72) Inventor: **Fujisawa, Yukio**
**31-104, 1 Mikagenakamachi 4-chome,**
**Higashinada-ku**
**Kobe, Hyogo 658(JP)**
Inventor: **Hinuma, Shuji**
**D73-207, 18 Tsukumodai 5-chome**
**Suita, Osaka 565(JP)**
Inventor: **Mayumi, Aki**
**295, Ikejiri**
**Osaka-sayama, Osaka 589(JP)**
Inventor: **Yamamoto, Tatsuo**
**1-30, Nakashinjuku 1-chome**
**Kashiwa, Chiba 277(JP)**

(74) Representative: **von Kreisler, Alek,**
**Dipl.-Chem. et al**
**Patentanwälte Von Kreisler-Selting-Werner,**
**Deichmannhaus am Hauptbahnhof**
**W-5000 Köln 1(DE)**

(54) **Expression plasmids and use thereof.**

(57) An expression plasmid is disclosed which comprises a DNA sequence coding for subunit B of a heat-labile enterotoxin, and a promoter selected from the group consisting of $\lambda P_L$ promoter, $\lambda P_R$ promoter, trp promoter, tac promoter and T7 promoter, the DNA sequence being located at a position downstream from the promoter. An Escherichia coli transformant bearing such a plasmid and the expression of subunit B of a heat-labile enterotoxin are also disclosed.

## EXPRESSION PLASMIDS AND USE THEREOF

### BACKGROUND OF THE INVENTION

This invention relates generally to a recombinant DNA for use in the production of subunit B (hereinafter referred to as LTB for brevity) of a heat-labile enterotoxin (hereinafter referred to as LT for brevity). More specifically, the present invention is concerned with an expression plasmid comprising a DNA coding for LTB, with a transformant containing the expression plasmid and with the production of LTB using the transformant.

Heat-labile enterotoxins (LT) are a kind of enterotoxins produced by enterotoxigenic Escherichia coli causing diarrhea. Among the LT produced by enterotoxigenic Escherichia coli , there are LTp produced by strains causing bacterial diarrgea in cattle and LTh produced by strains causing bacterial diarrhea in human [Honda, T. et al., Infect. Immun. , 34 , 337 (1981); Geary, S. J., ibid 36 , 215 (1982); Tsuji, T. et al., ibid 38 , 444 (1982)].

LT is composed of subunit A (hereinafter referred to as LTA for brevity) with a molecular weight of 28,000 and subunit B (LTB) with a molecular weight of 11,500 [Clements, J. D. & Finkelstein, R. A., Infect. Immun. , 24 , 760 (1979)]. One molecule of LT is composed of one molecule of LTA and five molecules of LTB. LTA has toxin activity while LTB functions to adhere to cell receptors [Gill, D. M. et al., ibid 33 , 677 (1981)].

The nucleotide sequences of the LTA gene and the LTB gene of LTh and LTP have been determined [Dallas, W. S. & Falkow, S., Nature , 277 , 406 (1979); Yamamoto, T & Yokota, T., J. Bacteriol ., 155 , 728 (1983); Yamamoto, T. et al., J. Biol. Chem. , 259 , 5037 (1984); Leong, J. et al., Infect. Immun. , 48 , 73 (1985)], from which LTA and LTB are presumed to have 240 amino acid residues and 103 amino acid residues, respectively.

Because of the similarity of LT to choleratoxin (hereinafter referred to as CT for brevity) with respect to physicochemical and immunological behaviors, the function and mechanism of LT are considered to be the same as those of CT, though details of LT have not yet been reported. Thus, LT is considered to cause ADP-ribosylation of regulatory protein Ns of adenylate cyclase so that the GTP hydrolysis activity of Ns is inactivated, with the result that adenylate cyclase is maintaned in an activated state. However, still unknown are further details of biochemical reaction mechanisms through which the activation of adenylate cyclase and the resulting increase of intracellular CAMP concentration cause such secretion of a large amount of water in intestinal canal as to lead to diarrhea.

It was recently revealed that CT and subunit B thereof (hereinafter referred to as CTB for brevity) serve to function as an adjuvant in mucous membranes of intestinal canals or respirators. Namely, CT and CTB can make it easy for other antigens to enter the mucous membrane and can facilitate the reactions in an early stage of sensitization in the immune system of topical mucous membranes [Tamura, S. I., Microbiol. Immunol. , 32 , 1145 (1988)]. Because of the similarity to CT, LT or LTB is expected to have similar functions.

It is reported that Escherichia coli having introduced thereinto a plasmid for Escherichia coli into which plasmid the entire coding region of LT, the coding region of LTA or the coding region of LTB has been inserted can secrete a portion of LT and a greater part of LTB, and cannot secrete LTA at all [Yamamoto, T. et al., J. Bacteriol. , 145 , 850 (1981); ibid 148 , 983 (1981); ibid 150 , 1482 (1982)]. In these reports, the LTB gene is expressed using the promoter of pBR322 or transcripted as part of the operon of the kanamycin-resistant gene in pACYC177 or chloramphenicol-resistant gene in pGA24. The expressed amount of LT and its subunits in these methods, however, was insufficient.

Since LTB is expected to serve to function as an adjuvant for coexistent antigens and to induce IgA in topical mucous membranes, there is a great demand for a large-scale production of LTB. In particular, LTB is expected to provide pharmacological effect in vaccine therapy against infection of pathogenic organisms through mucous membranes.

### SUMMARY OF THE INVENTION

It is, therefore, the prime object of the present invention to provide an expression plasmid and Escherichia coli transformed therewith useful for producing LTB.

There is provided in accordance with the present invention an expression plasmid comprising a DNA sequence coding for subunit B of a heat-labile enterotoxin, and a promoter selected from the group

consisting of $\lambda P_L$ promoter, $\lambda P_R$ promoter, trp promoter, tac promoter and T7 promoter, the DNA being located at a position downstream from the promoter.

In another aspect, the present invention provides an Escherichia coli transformant transformed with the above expression plasmid.

The present invention also provides a process for the production of subunit B of a heat-labile enterotoxin, comprising cultivating in a culture medium an Escherichia coli transformant transformed with the above expression plasmid, producing and accumulating the subunit B in a culture, and collecting the subunit B.

The present invention further provides a purifying method for subunit B of a heat-labile enterotoxin, applying the subunit B obtained by the above method to a purifying process comprising cationic exchange chromatography and gel filtration.

BRIEF DESCRIPTION OF THE DRAWINGS

Other objects, features and advantages of the present invention will become apparent from the detailed description of the invention which follows, when considered in light of the accompanying drawings, in which:

Fig. 1 shows the construction of the expression plasmid pLTB101 discussed in Example 1; and

Fig. 2 shows the results of electrophoresis showing expression of the transformant Escherichia coli MM294(DE3)/pLTB101.

Figs. 3, 4, 5 and 6 are graphs showing the course of purification of LThB in Example 3, showing the results of column chromatography (Fig. 3), the results of electrophoresis analyses by silver staining (Fig. 4-A) and by Western blotting (Fig. 4-B), the results of gel filtration (Fig. 5) and the results of electrophresis analyses (Fig. 6).

Fig. 7 shows the binding ability of LThB purified in Example 3 to some gangliosides.

Fig. 8 is a graph showing the result of reverse phase high performance liquid chromatography of the purified LThB.

DETAILED DESCRIPTION OF THE INVENTION

As the DNA sequence coding for LTB, there may be used any DNA as long as it codes for an LTB produced by enterotoxigenic Escherichia coli . Such a DNA may be a naturally occuring one or a chemically synthesized one. Further, the DNA may be the entire sequence of the LTB gene or may be a functional portion thereof. One suitable example of such DNAs is that obtained by cleaving, with suitable restriction enzymes, plasmid pJYL2299 [Yamamoto, T. & Yokota, T., J. Bacteriol. , 143 , 652 (1980)] containing cloned LTh. By amplification of a plasmid, into which the above DNA is sub-cloned, in Escherichia coli , DNA coding for LTB may be obtained in a large amount. A functional portion of LTB is one that retains some LTB function, e.g., adherence to cell receptors. This portion includes additions, deletions and substitutions from a normal full length sequence.

As a vector into which the DNA coding for LTB is to be inserted, there may be used any vector or plasmid as long as it is replicable in Escherichia coli . For example, such a vector or vehicle may be obtained by inserting a promoter into plasmid pBR322 [Sutcliffe, J. G., Cold Spring Harbor Symposium , 43 , 77 (1979)].

The promoter is selected from the group consisting of $\lambda P_L$ promoter, $\lambda P_R$ promoter, trp promoter, tac promoter and T7 promoter. Above all, the use of T7 promoter is particularly suitable for reasons of its strong promoter activity. These promoters may be prepared through enzymatic treatments of the corresponding genes or chemically synthesized based upon the reported sequences.

Examples of suitable expression vectors include pET-3c [Rosenberg, A. H. et al., Gene , 56 , 125 (1987)], pTB281 [Taniyama, et al., Journal of the Takeda Research Laboratories , 45 , 136 (1986), pTRP801 [Fujisawa, Y. et al., Nucl. Acids Res. , 11 , 3581 (1983)] and pKK233-2 (Pharmacia LKB, Sweden).

The expression plasmid according to the present invention for expressing a DNA sequence coding for LTB can be obtained by inserting the DNA sequence coding for LTB into the expression vector at a position downstream from the promoter.

The construction of the expression plasmid according to the present invention may be effected in any conventional method, such as a method disclosed in "Molecular Cloning" (1982), Cold Spring Harbor Laboratory.

As a host microorganism to be transformed with the expression plasmid of the present invention, there may be mentioned Escherichia coli .

The transformation of Escherichia coli with the expression plasmid (recombinant DNA) may be performed according to a method disclosed in, for example, Proc. Natl. Acad. Sci. U.S.A. , 69 , 2110 (1972) or Gene , 17 , 107 (1982)].

The resultant transformant is cultivated by any suitable known method using a liquid culture medium. The liquid medium contains a carbon source, a nitrogen source, an inorganic substance, and the like, required for the growth of the transformant. The carbon source may be, for example, glucose, dextrin, soluble starch or sucrose. The nitrogen source may be, for example, an inorganic or organic nitrogen source such as ammonium salt, a nitrate, corn steep liquor, peptone, casein, meat extract, soybean meal or potato extract liquor. The inorganic substance may be, for example, calcium chloride, sodiumdihydrogen phosphate or magnesium chloride. A yeast, a vitamin and a growth promoting factor may also be added to the medium. The pH of the medium is preferably in the range of about 6 to 8.

A preferred medium is LB medium [Molecular Cloning (1982), Cold Spring Harbor Laboratory] or M9CA medium containing glucose and Casamino acid [Miller, Journal of Experiments in Molecular Genetics , 431-433, Cold Spring Harbor Laboratory, New York (1972)]. These media may be supplemented with a suitable chemical such as isopropylthiogalactoside (IPTG) for the purpose of improving the action of the promoter.

The cultivation is generally carried out at about 15 to 43° C for about 3 to 48 hours with, if necessary, aeration and agitation.

From the above culture, LTB may be isolated in the following manner:

For extracting LTB from the cells, the cells are disrupted by any suitable method such as an ultrasonic oscillation method, a method using a French press, a mechanical method using glass beads or abrasion devices or a method using a lysozyme. A surfactant such as Triton X-100 or deoxycholate may be used if necessary. From the resulting extract or from the supernatant, LTB is collected and purified by any suitable method such as dialysis, ultrafiltration, isoelectric point precipitation, ion-exchange chromatography, gel filtration (gel permeation chromatography), affinity chromatography using anti- choleratoxin subunit B antibody or anti-LTB antibody, hydrophobic chromatography and reverse phase liquid chromatography. Especially preferred for the separation and purification of LTB is a method using a combination of ion exchange chromatography and gel filtration. As fillers for the ion exchange chromatography, cationic exchange resins containing a carboxyl-group as an exchanging group such as ST-Toyopearl 650 M (Toyo Soda, Japan) is preferable. Sovents for equilibriation of columns include 20 mM phosphate buffer (pH 6.0), and eluate solvents include 20 mM phosphate buffer containing 0 to 1 M NaCl (pH 6.0). As fillers for gel filtration, porous particles such as Sephacryl S-100HR (TM) capable of fractionating proteins having molecular weights of $1 \times 10^3$ to $1 \times 10^5$ are preferably used. As solvents for gel filtration, 5 to 20 mM phosphate buffer containing 0.5 to 1.5 % NaCl (pH 6.8 to 7.2) is preferably used. The LTB obtained is substantially pure. Preferably it has a purity of at least about 95%, more preferably, at least about 99%. This LTB is substantially free of other pyrogens and endotoxins.

LTB obtained by the present invention, which has the same activity as LTB obtained from enterotoxigenic Escherichia coli , can be used as an adjuvant.

LTB can be mixed with a foreign or human derived bioactive peptide or protein [e.g. hormone (insulin, human growth hormone, gonadotropin, inhibin, prolactin or the like), enzyme (serratiopeptidase or the like), cytokine [interferon (IFN), IFN-α, IFN-β, IFN-γ, interleukin (IL), IL-1, IL-2, IL-3, IL-4, IL-5, IL-6, IL-7, granular colony stimulating factor, granular macrophage colony stimulating factor, macrophage colony stimulating factor, erythropoietin or the like), growth factor or the like)] or a drug of low melucular weight, and administered pernasally, so that the absorption of the drug from nasal mucosa can be enhanced.

When bases, amino acids and so on are indicated by the abbreviations in this specification and the drawings, the abbreviations adopted by IUPAC-IUB Commission on Biochemical Nomenclature or commonly used in the art are employed. For example, the following abbreviations are used. When the optical isomer is capable of existing with respect to the amino acids, the L-form is represented unless otherwise specified.

DNA : Deoxyribonucleic acid

A : Adenine

T : Thymine

G : Guanine

C : Cytosine

EDTA : Etylenediaminetetraacetic acid

SDS : Sodium dodecyl sulfate

IPTG : Isopropylthiogalactoside

Gly : Glycine (G)

Ala : Alanine (A)

Val : Valine (V)

Leu : Leucine (L)

Ile : Isoleucine (I)

Ser : Serine (S)

Thr : Threonine (T)

Cys : Cysteine (C)

1/2 Cys: Half cysteine

Met : Methionine (M)

Glu : Glutamic acid (E)

Asp : Aspartic acid (D)

Lys : Lysine (K)

Arg : Arginine (R)

His : Histidine (H)

Phe : Phenylalanine (F)

Tyr : Tyrosine (Y)

Trp : Tryptophan (W)

Pro : Proline (P)

Asn : Asparagine (N)

Gln : Glutamine (Q)

$Ap^r$ : Ampicillin-resistant gene

$Tc^r$ : Tetracycline-resistant gene

The present invention will hereinafter be described in detail with the following Examples. It is understood of course that these Examples are merely illustrative and are not intended to limit the scope of the invention.

Transformant Escherichia coli MM294(DE3)/pLTB101 obtained in Example 2 was deposited with the Fermentation Research Institute, Agency of Industrial Science and Technology, Ministry of International Trade and Industry, Japan (FRI) under the accession number FERM BP-2583 on September 4, 1989. This microorganism was also deposited with the Institute for Fermentation, Osaka, Japan (IFO) under the accession number IFO 14933 on August 28, 1989.

Example 1

Construction of Expression Vector

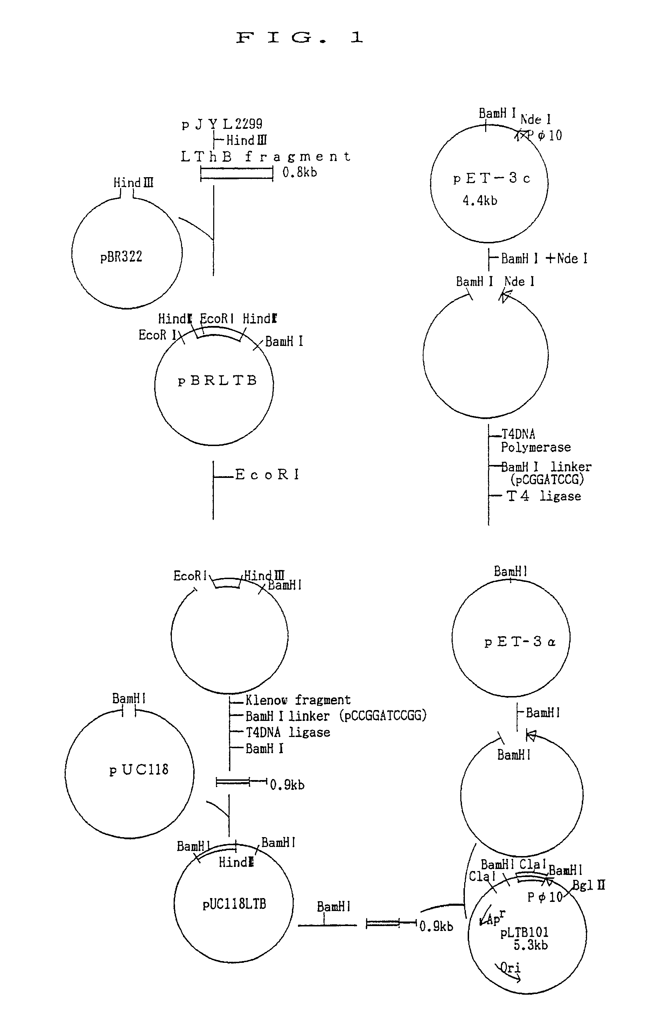

Plasmid pJYL2299 [Yamamoto, T., et al., J. Bacteriol. , 148 , 983 (1981)] containing the entire gene of human labile enterotoxin (LTh) was digested with HindIII to obtain a DNA fragment of 0.8 kb containing the entire coding region of subunit B (LThB) of LTh. This fragment was inserted into plasmid pBR322 at a HindIII site to obtain plasmid pBRLTB (Fig. 1). The plasmid was digested with EcoRI, and the resulting DNA was changed to flush ends with Klenow fragment (Takara Shuzo K.K. Japan). To the resulting DNA was then added BamHI linker (pCCGGATCCGG) (Takara Shuzo K.K. Japan) using T4 DNA ligase (Takara Shuzo K.K. Japan). The product was treated with BamHI to obtain a DNA fragment of about 0.9 kb, which fragment was subsequently inserted, for subcloning purpose, into plasmid pUC118 at the BamHI site, therby to obtain plasmid pUC118LTB.

Expression vector pET-3c [Rosenberg, A. H. et al., Gene , 56 , 125 (1987)] containing T7 promoter was digested with NdeI and BamHI and then treated with T4 DNA polymerase (Takara Shuzo K.K. Japan). To the resulting flush end DNA was ligated BamHI linker (pCGGATCCG) (Takara Shuzo K.K. Japan) using T4 DNA ligase to obtain plasmid pET-3d having BamHI cloning site. Inserted into plasmid pET-3d was a DNA fragment of 0.9 kb which had been obtained by digesting the above pUC118LTB with BamHI and which contained the entire coding region of LThB, thereby to obtain expression vector pLTB101 (Fig. 1). The fact that the DNA fragment had been inserted in a proper direction with respect to the promoter was confirmed by the fact that a DNA fragment of about 1.2 kb was obtained when this vector plasmid was digested with ClaI. The analogous plasmid having the 0.9 kb fragment inserted in the reverse direction was also used as a control in the followig experiment.

Example 2

Preparation of Transformant and Expression Thereof

Escherichia coli MM294(DE3), which had been obtained by integrating λphage DE3 [Studier, E. W. et al., J. Mol. Biol., 189, 113 (1986)] containing the RNA polymerase gene of T7 phage, was transformed with expression vector pLTB101 obtained in Example 1 to obtain transformant Escherichia coli MM294(DE3)-/pLTB101.

Clones of transformant Escherichia coli MM294(DE3)/pLTB101 were each cultivated at 37°C for 16 hours in an LB culture medium (5 ml) containing 100 μg/ml ampicillin. The culture liquid (0.1 ml) was then added into a 200 ml flask containing 10 ml of the above culture medium and incubated at 37°C. When the Klett value of 170 to 220 was reached, IPTG was added to a concentration of 0.1 mM and incubation was further continued at 30°C for 3 hours. The resulting culture solution were separated into cells and a supernatant. To the supernatant was added trichloroacetic acid (TCA) to a concentration of 10% to effect precipitation. The TCA precipitates from 4ml culture supernatant and the cells from 250 μl culture were each suspended in 50 μl sample buffer (50 mM Tris-HCl, pH 6.8 - 2 mM EDTA - 1% SDS - 1% mercaptoethanol -8% glycerol - 0.025% bromophenol blue). Each suspension was heated at 100°C for 5 minutes and then electrophoresed on 17.5% polyacrylamide gel.

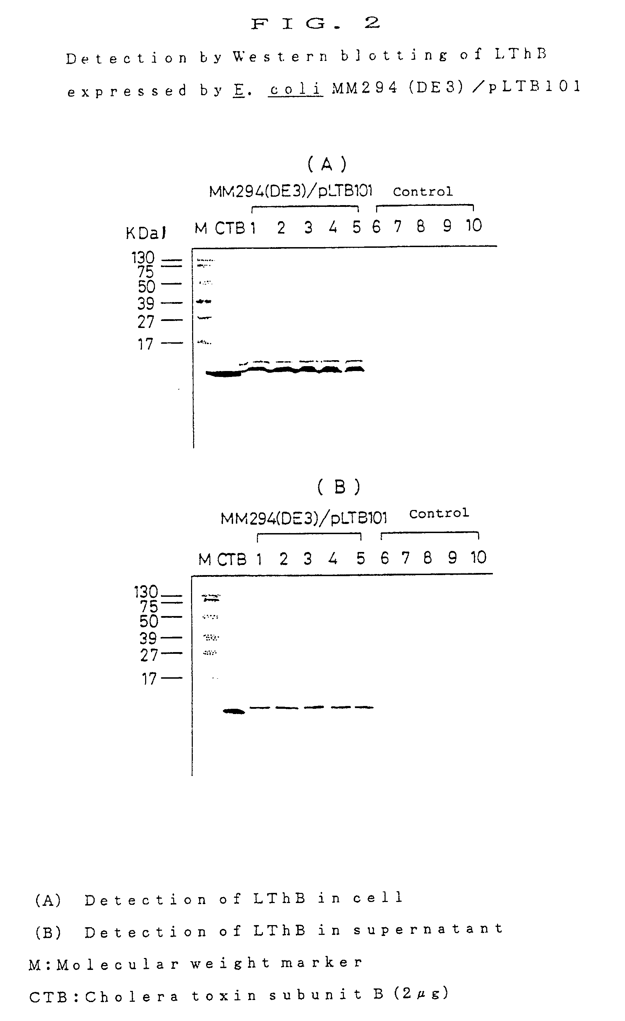

After electrophoresis, the products on the gel was transferred to a nitrocellulose filter to carry out Western blotting using goat anti-choleratoxin antibody (List Biological Labs., Inc.) as a primary antibody and peroxidase-labeled rabbit anti-goat IgG antibody (Kappel Products) as a secondary antibody. As a result, as shown in Fig. 2, there were observed bands specific to Escherichia coli MM294(DE3)/ pLTB101 at a position of about 1.2 - 1.4 kilodaltons in both cases of the cells (A) and the supernatant (B), indicating the expression of LThB.

Example 3

Purification of LThB

An LB medium (40 ml) containing 100 μg/ml ampicillin was inoculated with the transformant E. coli MM294(DE3)/pLTB101 obtained in Example 2, and it was cultivated overnight with shaking in a 200 ml flask at 37°C. The culture solution (10 ml) was added to 200 ml of M9CA medium containing 100 μg/ml ampicillin, followed by cultivation in a 1 liter flask at 37°C for 3.5 hours. IPTG was added to the culture solution to a final cocncentration of 0.4 mM, followed by further shaking culture overnight. The culture solution was centrifuged at 11,000 rpm by Serval RC-5B centrifuge equipped with SS34 roter, at 4°C for 20 minutes to obtain the supernatant. The above procedures were repeated to obtain 5 liter of the supernatant in total.

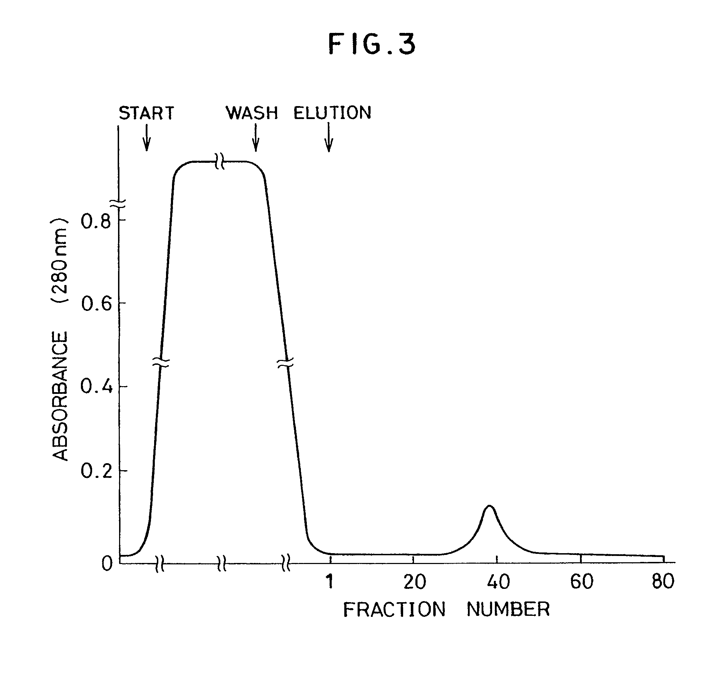

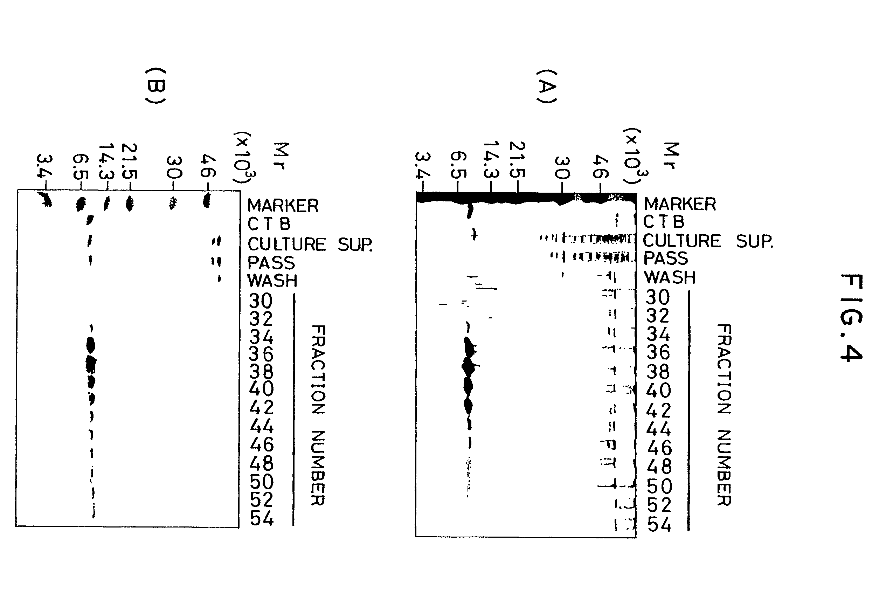

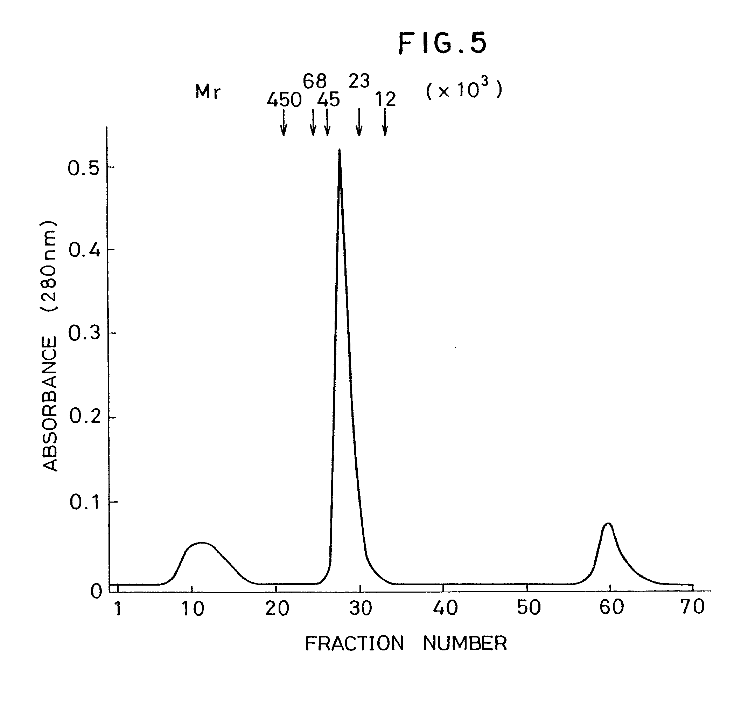

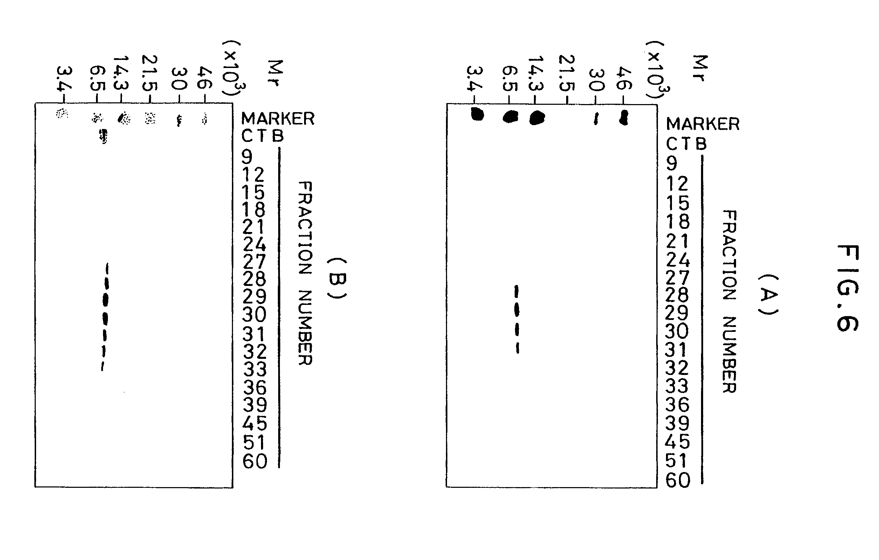

The supernatant was applied onto CM-Toyopearl 650M column (bed capacity: 520 ml, ∅ 4.2 x 37.5 cm) equilibrated with 20 mM phosphate buffer (pH 6.0), followed by washing of the column with the same buffer. Then, elution was carried out with the same buffer containing 0.5 M NaCl to be fractionated (12 ml/fraction) as shown in Fig. 3. The eluted LThB fractions were subjected to SDS-PAGE, and the product was identifed by silver staining (Fig. 4-A) and by Western blotting (Fig. 4-B). The fractions (fraction Nos. 35 to 44) were pooled and concentrated to 5 ml by ultrafiltration, and the concentrate was applied onto Sephacryl S-100 HR column (bed capacity: 130 ml, ∅ 1.6 x 65 cm) equilibrated with 10 mM phosphate buffer containing 0.85% NaCl (Fig. 5). The eluted LThB fractions were subjected to SDS-PAGE and the product was analyzed by silver staining and Western blotting (Fig. 6). LThB was eluted at a position of 35,000 of a molecular weight and it suggests that LThB may form trimer under the conditions. The eluted LThB fractions (fraction Nos. 27 to 32) were collected and concentrated by ultrafiltration to obtain a purified sample (2.4 mg/4 ml) having a purity of more than 99%.

Example 4

Properties of Purified LThB

6

The purified LThB obtained in Example 3 was eluted at a position of 12,000 of a molecular weight under reducing conditions by SDS-polyacrylamide gel electrophoresis.

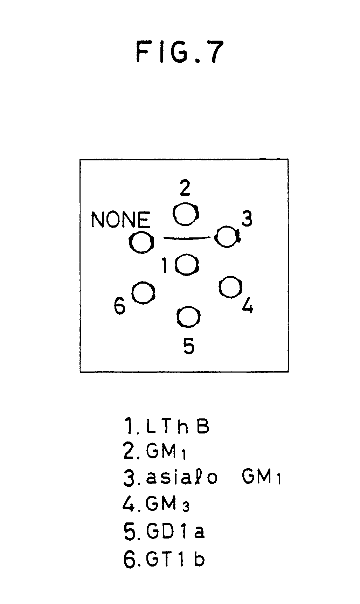

Binding ability of the purified LThB to some gangliosides was assayed according to a method of Holmgren et al. [ Infection and Immunity , 8 , 208-214 (1973)]. After an agar plate (1.2%) was punched to make holes (∅ 4 mm) as shown in Fig. 7, to each hole were added 6 μg of LThB and 10 μg of a ganglioside selected from the group of GM₁, asialo GM₁, Gm₃, $G_{DIa}$ and $G_{TIb}$ in 10 mM phosphate buffer containing 0.85 % NaCl (pH 7.0, 10 μl), and they were incubated. As a result, the purified LThB was bound specifically to monosialo ganglioside GM₁ to form a precipitate line (Fig. 7).

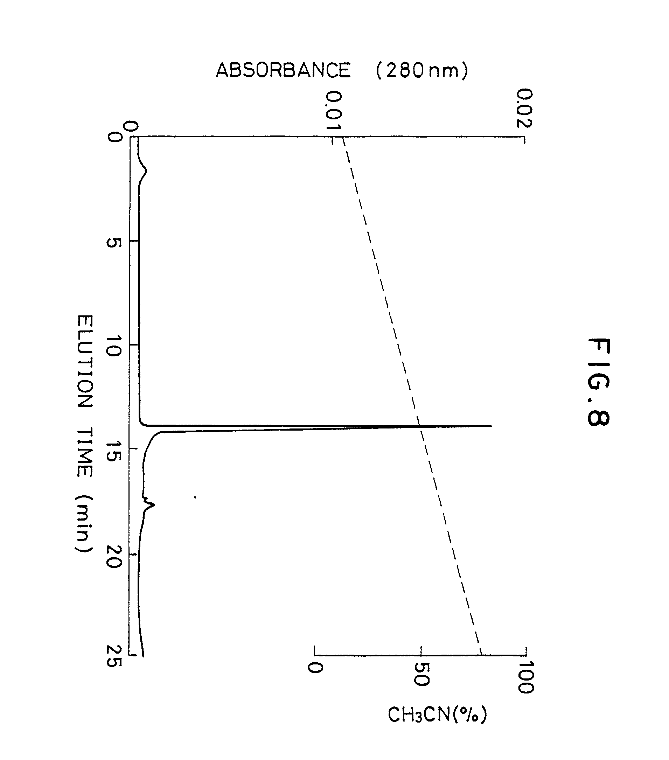

For analysis of an amino acid composition and a terminal sequence of LThB, reverse phase high performance liquid chromatography (RP-HPLC) was carried out as a pretreatment. The purified LThB (3.5 μg/6 μl) was adsorbed to Beckman Ultrapore RPsc C3 column (∅ 0.46 X 7.5 cm) equilibrated with distilled water containing 0.1% trifluoroacetic acid and 10% acetonitile, and then LThB was eluted with acetonitrile gradient from 10% to 80%. LThB was eluted at a position of about 50% of acetonitrile (Fig. 8). After drying the eluate fractions, the dried product was subjected to analysis of an amino acid composition and of a terminal sequence. Amino acid seqeunce was determined by PICO-TAG amino acid sequencer. The values approximately agreed with those deduced from the DNA sequence (Table 1).

N-terminal amino acid sequence was analysed according to automatic Edman degradation method using a Gas-phase Protein Sequenator (Applied Biosystems, Inc. model 470A). Phenylthiohydantoin-amino acid (PTH-amino acid) was identified by HPLC using Micropack SP-0 DS colomn (Varian Associates, Inc.). PTH-amino acids detected at each step are shown in Table 2. The amino acid sequence of 20 amino acid residues from the N-terminus agreed with that deduced from the DNA sequence. C-terminal amino acid residue was determined as asparagine by the hidrazine method, the result agreed with the C-terminus deduced from the DNA sequence.

Table 1

| Amino Acid Composition of the Purified LThB | | |
|---|---|---|
| Amino acid | Amino acid residue number per one molecule | |
| | Found | Deduced from the DNA sequence |
| Asp/Asn | 10.4 | 10 |
| Glu/Gln | 13.9 | 13 |
| Ser | 8.2 | 9 |
| Gly | 3.1 | 3 |
| His | 1.0 | 1 |
| Arg | 4.2 | 4 |
| Thr | 10.6 | 11 |
| Ala | 5.7 | 6 |
| Pro | 2.8 | 3 |
| Tyr | 3.9 | 4 |
| Val | 3.4 | 4 |
| Met | 3.8 | 4 |
| H-Cys | - | 2 |
| Ile | 11.3 | 12 |
| Leu | 5 | 5 |
| Phe | 1.9 | 2 |
| Trp | - | 1 |
| Lys | 8.8 | 9 |

Table 2

| N-terminal Amino Acid Sequence of the Purified LThB | | | |
|---|---|---|---|
| Cycle | PTH-amino acid residue | Detected amount (p mole) | Sequence expected from the DNA sequence |
| 1 | Ala | 1075 | Ala |
| 2 | Pro | 773 | Pro |
| 3 | Gln | 595 | Gln |
| 4 | Ser | 242 | Ser |
| 5 | Ile | 276 | Ile |
| 6 | Thr | 232 | Thr |
| 7 | Glu | 343 | Glu |
| 8 | Leu | 310 | Leu |
| 9 | - | | Cys |
| 10 | Ser | 95 | Ser |
| 11 | Glu | 242 | Glu |
| 12 | Tyr | 201 | Tyr |
| 13 | Arg | 399 | Arg |
| 14 | Asn | 196 | Asn |
| 15 | Thr | 98 | Thr |
| 16 | Gln | 165 | Gln |
| 17 | Ile | 122 | Ile |
| 18 | Tyr | 139 | Tyr |
| 19 | Thr | 68 | Thr |
| 20 | Ile | 84 | Ile |
| 1.5 nano mole of LThB was used for the analysis. -: cannot be determined. | | | |

## Claims

1. An expression plasmid comprising a DNA sequence coding for subunit B of a heat-labile enterotoxin or functional portion thereof, and a promoter selected from the group consisting of $\lambda P_L$ promoter, $\lambda P_R$ promoter, trp promoter, tac promoter and T7 promoter, the DNA sequence being located at a position downstream from the promoter.

2. An expression plasmid according to claim 1, wherein said promoter is T7 promoter.

3. An Escherichia coli transformant transformed with an expression plasmid comprising a DNA sequence coding for subunit B of a heat-labile enterotoxin or functional portion thereof, and a promoter selected from the group consisting of $\lambda P_L$ promoter, $\lambda P_R$ promoter, trp promoter, tac promoter and T7 promoter, the DNA sequence being located at a position downstream from the promoter.

4. A transformant according to claim 3, wherein said promoter is T7 promoter.

5. A transformant according to claim 4, which has the characteristics of Escherichia coli MM294(DE3)-/pLTB101 (FERM BP-2583).

6. A process for the production of subunit B of heat-labile enterotoxin, comprising cultivating in a culture medium an Escherichia coli transformant transformed with an expression plasmid comprising a DNA sequence coding for subunit B of a heat-labile enterotoxin or functional portion thereof, and a promoter selected from the group consisting of $\lambda P_L$ promoter, $\lambda P_R$ promoter, trp promoter, tac promoter and T7 promoter, the DNA sequence being located at a position downstream from the promoter, producing and accumulating the subunit B in a culture, and collecting the subunit B.

7. A process according to claim 6, wherein said promoter is T7 promoter.

8. A process according to claim 7, wherein said transformant has the characteristics of Escherichia coli MM294(DE3)/PLTB101 (FERM BP-2583).

9. A method for purifying subunit B of heat-labile enterotoxin, comprising cultivating in a culture medium an Escherichia coli transformant transformed with an expression plasmid comprising a DNA coding for subunit B of a heat-labile enterotoxin or functional portion thereof, and a promoter selected from the group consisting of $\lambda P_L$ promoter, $\lambda P_R$ promoter, trp promoter, tac promoter and T7 promoter, the DNA being located at a position downstream from the promoter; producing and accumulating the subunit B in a culture; collecting the subunit B; and applying the collected subunit B to purifying process comprising cationic exchange chromatography and gel filtration.

SEQ ID NO: 1

SEQUENCE TYPE: nucleotide sequence

SEQUENCE LENGTH: 10 base pairs

STRANDEDNESS: double stranded

TOPOLOGY: linear

MOLECULE TYPE: other nucleic acid (synthetic DNA)

HYPOTHETICAL (Y/N); N

ANTI-SENSE (Y/N): N

PROPERTIES: linker

CCGGATCCGG

# F I G . 1

# F I G. 2

Detection by Western blotting of LThB

expressed by E. coli MM294 (DE3)／pLTB101

( A )

MM294(DE3)／pLTB101    Control

KDal    M CTB 1   2   3   4   5   6   7   8   9   10

130 ——
75 ——
50 ——
39 ——
27 ——
17 ——

( B )

MM294(DE3)／pLTB101    Control

M CTB  1   2   3   4   5   6   7   8   9   10

130——
75——
50——
39——
27——
17——

(A)  Detection of LThB in cell

(B)  Detection of LThB in supernatant

M:Molecular weight marker

CTB:Cholera toxin subunit B (2μg)

FIG. 3

# FIG.4

(A)

(B)

FIG.5

# FIG.6

## (A)

## (B)

# FIG.7

1. L T h B
2. $GM_1$
3. asialo $GM_1$
4. $GM_3$
5. GD 1 a
6. GT 1 b

EP 0 416 505 A2

FIG. 8